# EUROPÄISCHE PATENTSCHRIFT

(11) **EP 0 784 632 B1**
(45) Veröffentlichungstag und Bekanntmachung des Hinweises auf die Patenterteilung: **07.01.1999**
(21) Anmeldenummer: 95935890.4
(22) Anmeldetag: 02.10.1995
(51) Int. Cl.: C07K 14/755, A61K 38/37

(54) **VERFAHREN ZUR GEWINNUNG VON HOCHREINEM VON WILLEBRAND-FAKTOR**
PROCESS FOR EXTRACTING HIGH-PURITY VON WILLEBRAND FACTOR
PROCEDE POUR L'EXTRACTION DU FACTEUR WILLEBRAND A HAUTE PURETE

(30) Priorität: 04.10.1994 DE 4435485
(43) Veröffentlichungstag der Anmeldung: 23.07.1997
(73) Patentinhaber: IMMUNO Aktiengesellschaft, A-1221 Wien (AT)
(72) Erfinder: FISCHER, Bernhard, A-1120 Wien (AT); MITTERER, Artur, A-2304 Orth/Donau (AT); DORNER, Friedrich, A-1230 Wien (AT); SCHWARZ, Hans-Peter, A-1180 Wien (AT); TURECEK, Peter, A-1190 Wien (AT); EIBL, Johann, A-1180 Wien (AT)
(74) Vertreter: Kolb, Helga, Dr. Dipl.-Chem.
(86) Internationale Anmeldenummer: EP9503892
(87) Internationale Veröffentlichungsnummer: WO9610584

(56) Entgegenhaltungen:
- EP-A- 0 469 985
- WO-A-89/12065
- VOX SANGUINIS, Bd. 62, Nr. 1, Februar 1992 BASEL, CH, Seiten 1-11, M. BURDOUF-RADOSEVICH UND T. BURNOUF 'Chromatographic Preparation of a Therapeutic Highly Purified von Willebrand Factor Concentrate from Human Cryoprecipitate' in der Anmeldung erwähnt

## Beschreibung

Die Erfindung betrifft ein Verfahren zur Gewinnung eines hochreinen von Willebrand-Faktors (vWF). Weiterhin bezieht sich die Erfindung auf rekombinanten von Willebrand-Faktor (rvWF), der nach dem erfindungsgemässen Verfahren erhältlich ist, sowie auf eine pharmazeutische Zusammensetzung, die den rvWF enthält.

Bei der Blutgerinnung erfolgt die Umwandlung des flüssigen Blutes in den Blutkuchen, eine gallertige Masse, die die Abdichtung verletzter Blutgefässe durch Pfropfbildung bewirkt. Dabei erfolgt die Umwandlung des im Plasma vorhandenen löslichen Fibrinogens in den faseriggallertigen Gerinnungsstoff, das Fibrin, in einem mehrstufigen Prozess (sogenannte Blutgerinnungs-Kaskade), an dem mindestens 15 verschiedene, mit römischen Ziffern gekennzeichnete Blutgerinnungs-Faktoren beteiligt sind, von denen jeder, wenn er aktiviert ist, die jeweils nächste inaktive Stufe aktiviert.

Von den Gerinnungsfaktoren zirkulieren Calicum-Ionen, Fibrinogen und Prothrombin (Faktor II) ständig im Blut, andere werden durch Gewebeverletzung oder Kontakt mit Kollagen oder Phospholipiden aus Thrombocyten (Faktor XII) aktiviert. Unter den übrigen Blutgerinnungs-Faktoren befinden sich mehrere Serin-Proteasen, wie Kallikrein, Thrombin und die aktivierten Faktoren VII, IX, X und XI.

Die Thrombocyten setzen sich unter Anwesenheit von von Willebrand-Faktor (ein Bestandteil des Gerinnungs-Faktors VIII) an Kollagen des verletzten Bindegewebes durch Adhäsion fest. Sie verändern ihre Form und bilden Fortsätze aus, und zudem erleichtert ihre äussere Membran die Adhäsion weiterer Thrombocyten. Danach setzen sie verschiedene Substanzen aus ihren Granula frei, wodurch eine Gefässkonstriktion sowie die Anlagerung und die Aktivierung anderer Faktoren der plasmatischen Blutgerinnung bewirkt werden.

Dem von Willebrand-Faktor kommen in der normal ablaufenden Blutgerinnung direkte und indirekte Aufgaben zu. Er bindet in einem Komplex an den Faktor VIII. Dieser Komplex dient zur Stabilisierung des Faktor VIII. Dieser stabilisierte Faktor VIII hat dann wesentliche Cofaktorfunktion bei der Aktivierung von Faktor X. Der von Willebrand-Faktor greift aber auch direkt in die Blutgerinnung ein, indem er die Plättchenaggregation an verletzten Gefässen vermittelt.

Bei der Hämophilie (Bluterkrankheit) ist die Blutgerinnung durch Mangel an bestimmten plasmatischen Blutgerinnungs-Faktoren gestört. Bei der Hämophilie A beruht die Blutungsneigung auf einem Mangel an Faktor VIII bzw. einem Mangel an vWF, der einen wesentlichen Bestandteil des Gerinnungsfaktors VIII ausmacht. Die Behandlung der Hämophilie A erfolgt durch Ersatz des fehlenden Gerinnungsfaktors durch Faktorenkonzentrate aus Blutkonserven, z.B. durch intravenöse Infusion von Faktor VIII, eines vWF Faktor VIII-Komplexes oder von vWF.

Es gibt mehrere Krankheitsbilder, die auf Unter- oder Überproduktion des von Willebrand-Faktors zurückzuführen sind. So führt z.B. eine Überproduktion von vWF zur vermehrten Neigung von Thrombosen, wohingegen eine Unterversorgung mit vWF eine vermehrte Blutungsneigung oder verlängerte Blutungszeit zur Folge hat.

Das von Willebrand-Syndrom kann sich in mehreren Erscheinungsformen manifestieren. Alle Formen zeichnen sich durch eine verlängerte Blutungszeit aus, die durch ein absolutes Fehlen eines funktionellen vWF begründet sein können. Der Mangel an vWF kann auch eine phänotypische Hämophilie A hervorrufen, da der vWF ein wesentlicher Bestandteil des funktionellen Faktor VIII ist. In diesen Fällen ist die Halbwertszeit des Faktor VIII derart verringert, dass er seine speziellen Funktionen in der Blutgerinnung nicht wahrnehmen kann.

Im Plasma zirkuliert der vWF in einer Konzentration von 5-10 mg/l in Form eines nicht-kovalenten Komplexes mit dem Faktor VIII. Der vWF ist ein Glycoprotein, welches in verschiedenen Zellen des menschlichen Körpers gebildet und später in die Zirkulation freigesetzt wird. Dabei wird in den Zellen ausgehend von einer Polypeptidkette mit einem Molekulargewicht von ca. 220000 (vWF-Monomer) durch Ausbildung von mehreren Schwefelbrücken ein vWF-Dimer (primäres vWF-Dimer) mit einem Molekulargewicht von ca. 550000 gebildet. Aus den vWF-Dimeren werden dann durch Verknüpfung weitere Polymere des vWF mit immer höheren Molekulargewichten, bis zu ca. 20 Millionen, hergestellt. Es wird vermutet, dass insbesondere den hochmolekularen vWF-Fraktionen eine essentielle Bedeutung bei der Blutgerinnung zukommt.

In der Literatur sind verschiedene Verfahren zur Reinigung und Konzentrierung von vWF beschrieben, die alle humanes Blutplasma als Quelle für den von Willebrand-Faktor verwenden.

Mit einem hochauflösenden elektrophoretischen Verfahren kann eine Strukturanalyse des vWF vorgenommen werden. Dabei wurde von Baillod et al., Thrombosis Research 66, 745-755, 1992, gefunden, daß die vWF-Multimeren in Banden aufgetrennt werden und jede Multimerenbande ein oder mehrere Satellitenbanden mit sich führt. Dieses Erscheinungsbild wird auf den proteolytischen Abbau von vWF-Multimeren zurückgeführt. Eine einfache Zugabe von Proteaseinhibitoren zu Blutproben konnte diesen Abbau nicht verhindern.

Ein abnormaler vWF vom Typ IIA zeigt ein verändertes Elektrophoresemuster. Als Resultat der Multimerenanalyse wird bei Patienten mit von Willebrand-Krankheit vom Typ IIA gefunden, daß die Multimeren jeweils als Einzelbanden (Singuletts) auftreten und nicht in Satellitenbanden aufgespalten werden. Dies wird auf die Tatsache zurückgeführt, daß eine Protease-sensitive Bindung zwischen Tyr-842 und Met-843 in den Typ IIA Patienten möglicherweise nicht gespalten wird. Diese Patienten zeigen unterschiedliche Krankheitsbilder im Zusammenhang mit einer Blutungsneigung.

Ein ähnliches Bild der Multimerenbanden wird von Fischer et al., FEBS Letters 351 (1994) 345-348, für einen rekombinanten vWF beschrieben. Dieser rvWF wird von CHO-Zellen exprimiert und eine Multimerenanalyse vorgenommen. Im Gegensatz zu Plasma-vWF wurde keine Triplettstruktur beobachtet. Demzufolge liegt dieser rvWF als vollständig intaktes Protein vor, der proteolytisch nicht abgebaut ist.

Jedoch ist der rvWF noch keinem Behandlungsverfahren, wie einer Reinigung, Virusinaktivierung bzw. Virusabreicherung unterzogen worden. Dementsprechend lag noch keine pharmazeutische Präparation vor.

Die im Stand der Technik beschriebenen vWF-Präparate enthalten den vWF in proteolytisch abgebauter Form. Die Stabilität dieser Präparate ist dadurch begrenzt. Auch die Versuche zur Verhinderung der Proteolyse nach Abnahme einer Blutprobe mit entsprechenden Inhibitoren führten nicht zu einem vWF mit intakter Struktur.

Die EP-A-0503991 beschreibt die Reinigung von vWF aus humanem Kryopräzipitat durch drei aufeinanderfolgende chromatographische Schritte: 1.
Ionenaustauschchromatographie an DEAE (DEAE-Cellulose, Diethylaminoethyl-Cellulose)-Fraktogel und Elution des vWF durch 0,15 M NaCl; 2. nochmalige
Ionenaustauschchromatographie an DEAE-Fraktogel und Elution des vWF durch 0,17 M NaCl; 3. Affinitätschromatographie an Gelatine-Sepharose®. Als Puffersysteme werden Puffer verwendet, die Aminosäuren und Calciumionen enthalten.

M. Burnouf-Radosevich und T. Burnouf, Vox Sang 62 (1992) 1-11 beschreiben eine ähnliche chromatographische Reinigung von Plasma-vWF durch Kombination einer Ionenaustauschchromatographie an DEAE-Fraktogel mit einer Gelatine-Sepharose®-Filtration in einem Puffersystem, das Aminosäuren und Calciumionen enthält.

Die WO-A-8912065 beschreibt die Trennung von Plasma-Proteinen aus Plasma-Kryopräzipitaten durch Bindung der Proteine an DEAE-Fraktogel und stufenweise Elution durch steigende Zugabe von NaCl. Das Verfahren eignet sich insbesondere zur Gewinnung eines Faktor VIII-Konzentrats hoher Reinheit zur Behandlung von Hämophilie A, sowie zur Gewinnung von Konzentraten von Fibrinogen, vWF und Fibronectin. Die vWF enthaltende Fraktion wird vorzugsweise einer zweiten Chromatographie am gleichen Anionenaustauscher unter Verwendung eines Puffers, der Aminosäuren und Calciumionen enthält, unterworfen.

Die EP-A-0416983 beschreibt die Gewinnung des vWF-Faktor VIII-Komplexes aus menschlichem Plasma durch Ausfällung mit einer Kombination aus Bariumchlorid und Aluminiumhydroxid und anschliessende Anionenaustauschchromatographie an DEAE-Fraktogel.

Nach Harrison et al., Thrombosis Res. 50 (1988) 295-304 wird der vWF-Faktor VIII-Komplex durch Chromatographie an Dextransulphat-Agarose gereinigt.
Bei der Reinigung des vWF-Faktor VIII-Komplexes nach diesen Verfahren soll allerdings der Faktor VIII:C in hoher Reinheit erhalten werden.

Zur Behandlung der Hämophilie A werden deshalb auch immer besser gereinigte Faktor VIII:C-Konzentrate eingesetzt, die den vWF nicht oder nur noch in Spuren enthalten. Deshalb sind solche Präparate für die Behandlung eines vWF-Mangels nicht geeignet. Der Bedarf an reinem von Willebrand-Faktor-Konzentrat ist also sehr gross.

Die EP-A-0469985 und die US-A-5252710 beschreiben ein Verfahren zur Herstellung von vWF aus Plasma-Kryopräzipitat, der weitgehend frei von Faktor VIII ist, bei dem in einem ersten Reinigungsschritt vWF vom Faktor VIII getrennt wird, weil vWF nicht an der Anionenaustauschersäule gebunden wird, sondern nur der Faktor VIII. In einem zweiten Schritt wird dann die Salzkonzentration des nicht an den Anionenaustauscher gebundenen Materials wesentlich verringert und vWF an einen zweiten Anionenaustauscher gebunden und dann mit einer Lösung höherer Ionenkonzentration wieder eluiert.

Die DE-A-3904354 beschreibt die Herstellung eines vWF-Konzentrates aus Plasma-Kryopräzipitat durch Trennung des vWF vom Faktor VIII, wobei Faktor VIII an einen Ionenaustauscher gebunden wird, vWF jedoch nicht.

Die US-A-5252709 beschreibt ein Verfahren zur Trennung von Faktor VIII, vWF, Fibronectin und Fibrinogen aus humanem Plasma, wobei zuerst Faktor VIII, vWF und Fibronectin an einen Ionenaustauscher vom DEAE-Typ gebunden werden, und anschliessend mittels steigender Salzkonzentration vom Ionenaustauscher getrennt eluiert werden.

Obwohl diese Verfahren die Reinigung des vWF unter Abtrennung des Faktor VIII beschreiben, kann eine, wenn auch geringfügige Kontamination mit Faktor VIII bzw. mit anderen Blutplasmaproteinen nicht ausgeschlossen werden.

Alle vWF-Konzentrate, die durch Reinigung des Proteins aus humanem Blutplasma erhalten werden oder in Kontakt mit biologischem Material aus Säugetieren getreten sind, sind ausserdem potentiell mit dem Risiko behaftet, krankheitserregende Moleküle, wie z.B. Viren, vom Plasmaspender zu enthalten.

Die Aufgabe der vorliegenden Erfindung bezieht sich auf die zur Verfügungstellung eines effizienten, einfachen und sicheren Verfahrens zur Herstellung eines hochreinen von Willebrand-Faktors, der im wesentlichen frei ist von anderen Blutplasmaproteinen, und insbesondere frei ist von Faktor VIII.

Der Erfindung lag weiters die Aufgabe zugrunde ein pharmazeutisches Präparat zur Verfügung zu stellen, welches einen vWF enthält, dessen Stabilität gegenüber den bisher bekannten Präparaten verbessert ist.

Diese Aufgabenstellung wird mit dem Gegenstand der vorliegenden Erfindung gelöst.

Gegenstand der vorliegenden Erfindung ist ein Verfahren zur Gewinnung von hochreinem von Willebrand-Faktor, bei dem rekombinanter von Willebrand-Faktor (rvWF) durch eine Anionenaustauschchromatographie an einem Anionenaustauscher vom quaternären Aminotyp chromatographisch gereinigt wird.

Vorzugsweise wird der durch die Anionenaustauschchromatographie gereinigte rvWF weiterhin durch eine Affinitätschromatographie an immobilisiertem Heparin in einer Pufferlösung, bestehend aus Puffersubstanzen und gegebenenfalls Salz, chromatographisch gereinigt

Rekombinanter von Willebrand-Faktor wird aus dem zellfreien Kulturfiltrat transformierter, virusfreier, tierischer Zellen mittels Zellkulturtechnik gewonnen.

Bevorzugte Ausgestaltungen dieses Verfahrens sind Gegenstand der Ansprüche 2 bis 13.

Weiterer Gegenstand der vorliegenden Erfindung ist ein rekombinanter von Willebrand-Faktor, der frei von Blutplasmaproteinen, insbesondere frei von Faktor VIII ist, und der nach dem erfindungsgemässen Verfahren erhältlich ist. Dieser rekombinante von Willebrand-Faktor ist physiologisch aktiv.

Gegenstand der Erfindung ist ferner die Verwendung eines nach dem erfindungsgemässen Verfahren erhältlichen rvWf zur Behandlung von Hämophilie A und verschiedener Formen der von Willebrand-Disease. Weiterer Gegenstand ist auch die Verwendung dieses rekombinanten von Willebrand-Faktors zur Herstellung einer pharmazeutischen Zusammensetzung zur Behandlung von Hämophilie A und verschiedener Formen der von Willebrand-Disease.

Gegenstand der vorliegenden Erfindung ist ferner eine pharmazeutische Zusammensetzung gemäss Anspruch 18, die dadurch gekennzeichnet ist, dass sie den nach dem erfindungsgemässen Verfahren erhältlichen rvWF in einem physiologisch annehmbaren Träger enthält.

Ein weiterer Gegenstand der Erfindung ist eine pharmazeutische Präparation enthaltend den virussicheren rvWF, der aus Multimeren mit einer hohen strukturellen Integrität besteht.

Rekombinanter vWF (rvWF) wird aus zellfreiem Kulturmedium nach Fermentation tierischer Zellen isoliert und gereinigt. Das für die Fermentation verwendete Kulturmedium stellt eine für derartige Zwecke übliche komplexe, synthetische Mischung aller zum Leben der tierischen Zellen notwendigen Stoffe, wie Vitamine, Zucker, Salze, Hormone, Antibiotika und Puffersubstanzen, dar und unterscheidet sich daher in allen grundlegenden Eigenschaften wesentlich von der Zusammensetzung von menschlichem Blutplasma oder Plasma-Kryopräzipitat. Es war deshalb nicht vorauszusehen, dass sich das erfindungsgemässe Verfahren in hervorragender Weise zur Herstellung von hochreinem von Willebrand-Faktor eignen würde. Bei dem erfindungsgemässen Verfahren wird vorzugsweise ein rekombinantes vWF-Konzentrat aus zellfreien Kulturüberständen transformierter Zellen eingesetzt.

Im erfindungsgemässen Verfahren wird als Puffersystem eine Pufferlösung bestehend aus Puffersubstanzen und ggf. Salz, vorzugsweise Kochsalz, verwendet, die vorzugsweise frei von Stabilisatoren, Aminosäuren und anderen Zusätzen ist. Aus dem Stand der Technik ist bekannt, dass Stabilisatoren, Aminosäuren und andere Zusätze benötigt werden, um einerseits den von Willebrand-Faktor zu stabilisieren, andererseits den Faktor VIII - von Willebrand Komplex zu destabilisieren und auch die Abtrennung anderer Proteine zu erleichtern. Bei dem erfindungsgemässen Verfahren kann gänzlich auf solche Komponenten im Puffer verzichtet werden, wobei trotzdem ein physiologisch aktiver rekombinanter von Willebrand-Faktor erhalten wird.

Als Puffersystem wird vorzugsweise ein von Stabilisatoren, Aminosäuren und anderen Zusätzen freies Puffersystem, wie z.B. Tris-HCl/NaCl-Puffer, Phosphatpuffer, Citratpuffer verwendet.

Die Anionenaustauschchromatographie und/oder die Affinitätschromatographie werden vorzugsweise in einem pH-Bereich von 6,0 bis 8,5, und besonders bevorzugt bei einem pH-Wert von 7,4, durchgeführt.

Die Elution des bei der Anionenaustauschchromatographie an den Anionenaustauscher und bei der Affinitätschromatographie an immobilisiertes Heparin gebundenen rvWF erfolgt vorzugsweise durch Erhöhung der Salzkonzentration.

Als Anionenaustauscher vom quaternären Aminotyp wird vorzugsweise ein Fraktogel mit Tentakelstruktur und insbesondere EMD-TMAE-Fraktogel verwendet.

Vorzugsweise wird der rvWF an den Anionenaustauscher bei einer Salzkonzentration von < 270 mM gebunden, und bei einer Salzkonzentration > 270 mM, und vorzugsweise bei > 280 mM, eluiert. Als Salze sind lösliche ein- und zweiwertige Salze verwendbar, wobei NaCl bevorzugt wird.

Für die Affinitätschromatographie kann jeder Träger, an dem Heparin gebunden werden kann, verwendet werden. Als gut geeignet erwiesen sich zum Beispiel AF-Heparin-Toyopearl® (ein synthetisches grossporiges, hydrophiles Polymer auf der Basis von Methacrylat; Tosohaas), Heparin EMD-Fraktogel® (ein synthetisches hydrophiles Polymer auf der Basis von Ethylenglykol, Methacrylat und Dimethacrylat; Merck) oder Heparin Sepharose Fast Flow® (enthaltend natürliche Dextran- bzw. Agarosederivate; Pharmacia).

Vorzugsweise wird der durch die Stufe der Anionenaustauschchromatographie vorgereinigte rvWF an das immobilisierte Heparin bei einer Salzkonzentration < 150 mM gebunden, und bei einer Salzkonzentration von > 150 mM, vorzugsweise von 200 bis 300 mM und bevorzugter 160 mM bis 270 mM eluiert. Als Salz sind lösliche ein- und zweiwertige Salze verwendbar, wobei NaCl bevorzugt wird.

Aufgrund der Molekulargrösse des rvWF (Molekulargewicht von 500.000 bis mehrere Millionen) werden bei dem erfindungsgemässen Verfahren sowohl bei der Anionenaustauschchromatographie als auch bei der Affinitätschromatographie vorzugsweise solche Trägermaterialien, wie z.B. Gele mit Tentakelstruktur, verwendet, die das rvWF-Molekül in seiner Diffusion und Verteilung innerhalb der Trägerstruktur nicht behindern.

In einer bevorzugten Ausführungsform des Verfahrens gemäss der Erfindung wird das zellfreie Kulturmedium zuerst an einem starken Anionenaustauscher filtriert, wobei der rvWF durch den Austauscher gebunden wird. Als Anionenaustauscher wird vorzugsweise ein grossporiges Gel mit Tentakelstruktur und mit einer stark bindenden Ionenaustauschergruppe vom quaternären Amino-Typ, wie z.B. EMD-TMAE-Fraktogel, verwendet. Nach Entfernung von Begleitproteinen und Verunreinigungen mittels salzhaltigem, vorzugsweise NaCl-haltigem Puffer wird rvWF dann vom Ionenaustauscher in angereicherter Form eluiert. Im zweiten Reinigungsschritt der Affinitätschromatographie wird das den rvWF enthaltende Eluat mit einem Affinitätsträger mit kovalent gebundenem Heparin in Kontakt gebracht, wobei rvWF an diesen Träger bindet. Nach der Entfernung von Fremdsubstanzen und Fremdproteinen durch ein geeignetes Elutionsmittel (wie z.B. Puffersubstanz) wird der rvWf vom Affinitätsträger eluiert, vorzugsweise mittels eines NaCl enthaltenden Puffersystems.

Nach dem erfindungsgemässen Verfahren zur Gewinnung eines hochreinen von Willebrand-Faktors lässt sich auf effiziente und einfache Weise ein hochreiner rvWF erhalten, der frei von Antikörper, frei von Blutplasmaproteinen, und insbesondere frei von Faktor VIII, physiologisch aktiv und frei von pathogenen Viren ist.

Der hochreine rvWF ist des weiteren dadurch gekennzeichnet, dass der Anteil des vWF-Proteins zum Gesamtprotein mindestens 80 %, insbesondere mindestens 86 %, beträgt.

Der nach dem erfindungsgemässen Verfahren erhältliche hochreine rvWF kann aufgrund seiner Eigenschaft, dass er frei von Antikörper, frei von Plasmaproteinen, frei von pathogenen Viren und frei von Faktor VIII ist, bei der Behandlung von Hämophilie A gezielt eingesetzt werden.

Des weiteren kann der nach dem erfindungsgemässen Verfahren erhältliche hochreine rvWF zur Behandlung verschiedener Formen der von Willebrand-Disease verwendet werden.

Erfindungsgemäß wird weiters eine stabile pharmazeutische Präparation zur Verfügung gestellt, die einen rvWF enthält, der aus Multimeren mit einer hohen strukturellen Integrität besteht. Der rvWF ist derart stabil, daß er als virussicheres Präparat zur Verfügung gestellt werden kann. Die Virussicherheit ist durch Verfahrensschritte zur Behandlung des rvWF zur Inaktivierung von Viren bzw. Abreichung von Viren gewährleistet.

Zur Inaktivierung von Viren eignet sich insbesondere eine Hitzebehandlung in Lösung bzw. in festem Zustand, welche sowohl lipidumhüllte als auch nicht-lipidumhüllte Viren verläßlich inaktivieren kann. Beispielsweise wird die erfindungsgemäße Präparation gemäß der EP 0 159 311 in festem, nassen Zustand hitzebehandelt. Andere Verfahren zur Virus-Inaktivierung umfassen auch die Behandlung mit Detergenzien oder chaotropen Substanzen, beispielsweise gemäß der EP 0 519 901, WO 94/13329, DE 44 34 538, EP 0 131 740 und WO 90/15613.

Der rvWF ist in der erfindungsgemäßen Präparation vorzugsweise als hochreines Protein enthalten, welches durch ein chromatographisches Reinigungsverfahren erhalten wird. Die chromatographische Reinigung erfolgt insbesondere durch Ionenaustauschchromatographie und/oder Affinitätschromatographie. Dafür können u. a. Materialien zum Anionenaustausch, darunter synthetische Trägermaterialien oder Träger auf Kohlenhydratbasis, mit Liganden, wie DEAE-, TMAE-, QAE-, Q- oder Aminoalkylgruppen herangezogen werden, bzw. Träger mit immobilisierten Substanzen, die eine spezifische Affinität für vWF aufweisen. Geeignete Affinitätsmaterialien enthalten beispielsweise Heparin. Dieses Reinigungsverfahren eignet sich v. a. für die großtechnische Gewinnung des rvWF.

Dabei ist zu beachten, daß überraschenderweise der rvWF in der erfindungsgemäßen Präparation eine ausreichende Resistenz gegenüber einem proteolytischen Abbau aufweist, sodaß auf den Zusatz üblicher Stabilisatoren verzichtet werden kann. In Ausnahmefällen kann aber auch während des Herstellungsverfahrens ein entsprechender Proteaseinhibitor zugesetzt werden, um die intakte Struktur zu erhalten. Zur weiteren Unterstützung der Stabilität des vWF kann das pharmazeutische Präparat auch ein Polyalkylenglycol, wie PEG oder Polypropylenglycol, oder Glycerin in einer Konzentration enthalten, die den rvWF nicht präzipitiert und physiologisch verträglich ist.

Erfindungsgemäß zeigt der rvWF in dem Präparat nach elektrophoretischer Analyse Multimerenbanden in Abwesenheit von Satellitenbanden. Dies entspricht der Struktur eines vWF als nicht-fragmentiertes, also intaktes Protein. Vorzugsweise zeigt der rvWF im pharmazeutischen präparat das gesamte Spektrum an Multimeren, insbesondere auch den vWF mit hohem Molekulargewicht, ähnlich der nativen Multimerenverteilung.

Die Stabilität der erfindungsgemäßen Präparation ist vor allem für ein Flüssigpräparat erforderlich. Eine Lösung des erfindungsgemäßen Präparates ist beispielsweise bei Raumtemperatur mindestens 48 Stunden stabil, vorzugsweise mindestens 72 Stunden, und bei 4°C mehr als 2 Jahre lagerfähig. Die Stabilität zeigt sich durch einen nur unwesentlichen Aktivitätsverlust von weniger als 50%, vorzugsweise weniger als 20%, am meisten bevorzugt weniger als 10%. Damit eignet sich das erfindungsgemäße Präparat als Infusionspräparat, welches auch über eine Dauer von mehreren Stunden einen Patienten infundiert werden kann ohne Risiko der Veränderung des Präparates und der Notwendigkeit der Veränderung des Dosierungsschemas. Hinsichtlich der Vermeidung von möglichen Nebenwirkungsreaktion ist es gleichfalls vorteilhaft ein Protein mit intakter und stabiler Struktur zu verabreichen.

Es hat sich herausgestellt, daß das erfindungsgemäße pharmazeutische Präparat ohne Nebenwirkungsreaktionen, wie Thrombosebildung, Thrombozytenaktivierung oder Thrombozytopänie an einen Patienten verabreicht werden kann. Dies war vor allem deshalb überraschend, weil der rvWF im erfindungsgemäßen Präparat ein ähnliches Multimerenmuster aufweist wie die für die Typ IIA-von Willebrand-Krankheit verantwortliche Form.

Die Formulierung der erfindungsgemäßen pharmazeutischen präparation kann in an sich bekannter und üblicher Weise erfolgen, z.B. mit Hilfe von Salzen und gegebenenfalls Aminosäuren, aber auch in Gegenwart von Tensiden vorgenommen werden. Vorzugsweise werden Salze, wie z.B. Natriumchlorid oder Kalziumchlorid, verwendet und ein pH im Bereich von 6-8 gewählt. Als Aminosäuren sind Glycin oder Lysin bevorzugt. Ebenso kann ein pharmazeutisch akzeptabler Puffer gewählt werden. Aufgrund der hohen Stabilität des rvWF kann üblicherweise auf die Verwendung von Stabilisatoren, wie Trägerproteine oder Inhibitoren in der Formulierung verzichtet werden.

Die bevorzugte Konzentration des rvWF in der verabreichungsfertigen Lösung ist im Bereich von 1 bis 100 Einheiten/ml. Durch die hohe Reinheit des Präparates kann dieses auch in Konzentrationen bis zu 1000 E/ml formuliert werden. Die Aktivität ist durch die Ristocetin-mediierten Plättchenaggregation charakterisiert und wird als Ristocetin-Cofaktor-Aktivität (RCoF) angegeben (siehe dazu Journal of Clinical Investigation 52, 2708-2716, 1973). Die übliche Dosis für den vWF liegt im Bereich von 40-80 RCoF-Einheiten/kg in Intervallen von 6-48 Stunden. Als initiale Dosis kann auch eine höhere Dosis bis zu 200 RCoF gewählt werden.

Die Halbwertszeit des rvWF nach Verabreichung des erfindungsgemäßen Präparates ist überraschenderweise mit einer biologischen Halbwertszeit von mehr als 20 Stunden deutlich länger als für die Präparate des Stand der Technik.

Gemäß einer bevorzugten Ausführungsform wird der rvWF in einer Form erhalten, die nach Verabreichung an ein Säugetier das Multimerenmuster mit einer Singulettstruktur beibehält. Eine proteolytische Aufspaltung der Singuletts in Satellitenbanden bleibt somit aus.

Vorzugsweise enthält die erfindungsgemäße pharmazeutische Präparation den rvWF als einzigen wesentlichen Bestandteil. Somit kann diese Präparation im wesentlichen aus hochgereinigten rvWF bestehen.

Auch kann der nach dem erfindungsgemässen Verfahren erhältliche rvWF zur Stabilisierung von Faktor VIII, von rekombinant hergestelltem Faktor VIII oder funktionellen Deletionsmutanten von Faktor VIII verwendet werden, wobei die Stabilisierung in vitro nachgewiesen werden kann.

Ein so stabilisiertes Faktor VIII-Präparat ist nicht wie Plasmaprodukte mit dem Risiko behaftet, mit pathogenen Viren kontaminiert zu sein.

Überraschenderweise wurde gefunden, dass rvWF eine potentiell höhere Bindungskapazität an Faktor VIII besitzt und damit Faktor VIII effizienter bindet als plasmatischer vWF.

Gegenstand der vorliegenden Erfindung ist daher auch ein nach dem erfindungsgemässen Verfahren erhältlicher rvWF, dadurch gekennzeichnet, dass er erhöhte Bindungskapazität für Faktor VIII aufweist.

Zur Herstellung pharmazeutischer Präparationen werden die den hochreinen rekombinanten von Willebrand-Faktor enthaltenden Fraktionen vorzugsweise aufkonzentriert und das Konzentrat weiter verarbeitet.

Die pharmazeutischen Zusammensetzungen können in einer zur Behandlung von Hämophilie A und verschiedener Formen der von Willebrand-Disease üblichen und gebräuchlichen Darreichungsform vorliegen; vorzugsweise liegen sie in Form eines zur Infusion geeigneten Präparates vor.

In den folgenden Beispielen wird die Erfindung näher erläutert, ohne sie darauf zu beschränken.

Das Beispiel 1 beschreibt die Reinigung von aus zellfreiem Kulturmedium nach Fermentation transformierter tierischer Zellen erhaltenem rvWF durch Anionenaustauschchromatographie. Die weiterführende Reinigung durch die Verfahrensstufe der Affinitätschromatographie ist im Beispiel 2 beschrieben.

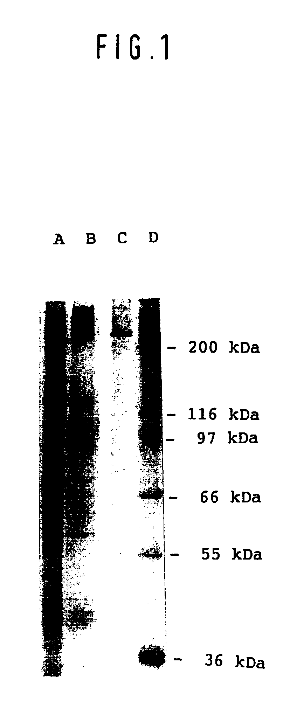

- Fig. 1: stellt eine 8%-ige SDS-PAGE Auftrennung von rvWF dar. Spur A: Kulturmedium; Spur B: Fraktion 280 mM NaCl nach dem Fraktogel; Spur C: 270 mM NaCl Fraktion nach der Heparin-Affinitätschromatographie; Spur D: Molekulargewichtsmarker.

### BEISPIEL 1

### Reinigung von rvWF aus Kulturüberständen durch Anionenaustauschchromatographie:

Rekombinanter vWF wurde gemäss üblicher Verfahren nach Infektion von Vero Zellen (Affen-Nierenzellen) mit Vaccinia-Virus in Zellkulturtechnik gewonnen. Vero/Vaccinia Expressionssysteme und Zellkulturbedingungen werden in F.G. Falkner et al., Thrombosis and Haemostasis 68 (1992) 119-l24, N. Barrett et al., AIDS Res. 5 (1989) 159-171 und F. Dorner et al., AIDS Vaccine Research and Clinical Trials, Marcel Dekker, Inc, New York (1990), ausführlich beschrieben. Die Expression von rvWF erfolgte in synthetischem DMEM-Standardmedium (Dulbeccos minimal essential medium).

Rekombinanter vWF kann auch durch Transformation von CHO-Zellen gewonnen werden.

Nach der Fermentation der transformierten Zellen wurde das Kulturmedium abgetrennt, und Zellen und Zellbruchstücke wurden durch Zentrifugation entfernt. Weitere kleinere Bestandteile, wie Membranbruchstücke oder Bakterien, wurden durch Filtration durch Filter mit einer Porengrösse von 0,4 µm entfernt.

770 ml zellfreier Kulturüberstand wurde mit einer Fliessgeschwindigkeit von 2 ml/cm2/min über eine Säule (1,6 cm x 5 cm, gefüllt mit 10 ml Anionenaustauscher EMD-TMAE-Fraktogel (Merck)) filtriert. Das Gel wurde zuvor mit 20 mM Tris-HCl Puffer (pH 7,4) equilibriert. Anschliessend wurde die Säule mit 20 mM Tris-HCl Puffer (pH 7,4) gewaschen.

Fremdstoffe wurden durch Waschen der Säule mit 200 mM NaCl enthaltendem Puffer entfernt. Der rvWF wurde dann mit 280 mM NaCl in 20 mM Tris-HCl Puffer (pH 7,4) vom Träger eluiert. Schliesslich wurde mit 1 M NaCl eventuell vorhandenes Restmaterial von der Säule eluiert. Während der Chromatographie wurde die Proteinabsorption in üblicher Weise bei 280 nm verfolgt. Nach der Chromatographie wurde die Proteinkonzentration nach der Bradford Methode (M. Bradford, Anal. Biochem. 72 (1976) 248-254) bestimmt. Der Gehalt an rvWF wurde mittels eines handelsüblichen ELISA Systems (Boehringer Mannheim) bestimmt.

Es wurde gefunden, dass nahezu der gesamte rvWF an den Träger gebunden wurde. rvWF wurde durch 0,28 M NaCl vom Anionenaustauscher eluiert. Die Ergebnisse der Reinigung von rvWF am Anionenaustauscher sind in Tabelle 1 zusammengefasst.

Durch die in diesem Beispiel beschriebene Reinigung wurde der rvWF um das 6-fache angereichert.

**TABELLE 1**

| Probe | Volumen (ml) | Gesamtprotein (µg/ml) | rvWF (µg/ml) | rvWF/ Gesamtprotein |
|---|---|---|---|---|
| Zellfreier Kulturüberstand | 770 | 113 | 7,9 | 0,069 |
| Elution mit 200 mM NaCl | 95 | 147 | 0,0016 | 0,00001 |
| Elution mit 280 mM NaCl | 75 | 168 | 61 | 0,36 |
| Elution mit 1 M NaCl | 50 | 196 | 6 | 0,03 |

### BEISPIEL 2

### Reinigung von rvWF durch Affinitätschromatographie:

Ein nach Beispiel 1 erhaltener rvWF wurde zur Verringerung der Salzkonzentration (160 mM NaCl) mit 20 mM Tris-HCl Puffer (pH 7,4) verdünnt. Anschliessend wurde die Lösung durch eine Säule (1,6 cm x 5 cm, gefüllt mit 10 ml AF Heparin Toyopearl 650 (Tosohaas)) mit einer Fliessgeschwindigkeit von 1 ml/cm2/min filtriert. Die Säule war zuvor mit 20 mM Tris-HCl Puffer (pH 7,4) equilibriert worden. Unspezifisch gebundene Proteine wurden zuerst durch Waschen mit 20 mM Tris-HCl Puffer (pH 7,4) entfernt. rvWF wurde durch 270 mM NaCl in 20 mM Tris-HCl Puffer (pH 7,4) vom Träger eluiert. Schliesslich wurde mit 1 M NaCl Restmaterial von der Säule eluiert. Während der Chromatographie wurde die Proteinabsorbtion in üblicher Weise bei 280 nm verfolgt. Nach der Chromatographie wurde die Proteinkonzentration mittels der Bradford Methode (M. Bradford, l.c.) bestimmt. Der Gehalt an rvWF wurde mittels eines handelsüblichen ELISA Systems (Boehringer Mannheim) bestimmt.

Es wurde gefunden, dass nahezu der gesamte rvWF an den Träger gebunden wurde. Bei der Elution mit 270 mM NaCl wurde der Grossteil des rvWF von der Säule eluiert, während die Waschung mit 1 M NaCl nur noch Spuren von rvWF enthält. Die Ergebnisse dieses Reinigungsschrittes sind in Tabelle 2 zusammengefasst. Durch diesen Reinigungsschritt wurde der Anteil des rvWF-Proteins zum Gesamtprotein auf über 86 % erhöht.

Mit einer denaturierenden SDS-Proteingelelektrophorese (U.K. Laemmli, Nature 227 (1970) 680-685) und anschliessendem Western-Blot wurde die Fraktion von 270 mM NaCl genauer untersucht.

Wie in Figur 1 dargestellt, ergab die denaturierende elektrophoretische Analyse, dass durch die im Beispiel 1 und 2 beschriebene Reinigung rvWF in hoher Reinheit gewonnen wurde. Im so gewonnenen Produkt konnten keine anderen Gerinnungsfaktoren, wie z.B. Faktor VIII, nachgewiesen werden.

**TABELLE 2**

| Probe | Volumen (ml) | Gesamtprotein (µg/ml) | rvWF (µg/ml) | rVWF/ Gesamtprotein |
|---|---|---|---|---|
| rvWf-Konzentrat | 225 | 50 | 13,9 | 0,27 |
| Elution mit 270 mM NaCl | 43 | 70 | 60 | 0,86 |
| Elution mit 1 M NaCl | 32 | 25 | 2 | 0,08 |

Der gereinigte rvWF besitzt eine Aktivität von 4,32 U/mg rvWF:Ag in bezug auf die Plättchenaggregation.

### BEISPIEL 3

Mittels Heparin Affinitätschromatographie wurden sowohl plasmatischer vWF (p-vWF), vWF aus Kryopräzipitat (k-vWF) als auch rekombinanter vWF (r-VWF) gereinigt. Die unterschiedlichen vWF-Präparate wurden auf ihre Bindung zu Faktor VIII untersucht.

**Tabelle 3**

| Probe | Stöchiometrie vWF : Faktor VIII |
|---|---|
| r-vWF | 2,0 : 1 |
| k-vWF | 2,6 : 1 |
| p-vWF | 3,0 : 1 |

Tabelle 3 zeigt die Daten der Stöchiometrie von vWF : Faktor VIII. Die Daten zeigen, das r-vWF eine wesentlich höhere Bindungskapazität für Faktor VIII besitzt als p-vWF.

### BEISPIEL 4

### Stabilität von rekombinantem von Willebrand Faktor in Lösung

Eine von Willebrand Faktor-Präparation wurde, wie in Beispiel 2 beschrieben, hergestellt und in einem Puffer, enthaltend 5 g/l Na₃Citrat.2H₂O, 2 g/l NaCl, 5 g/l Glycin, 5 g/l L-Lysin.HCl und 0,62 g/l CaCl₂.2H₂O, pH 7,0, so formuliert, daß die von Willebrand Faktor-Konzentration gemessen mittels Ristocetin-mediierter Plättchenaggregation 10 E/ml war. Eine derartige Lösung wurde bei 4 °C, 25 °C, 37 °C und 50 °C bis zu 70 Std gehalten. Zu verschiedenen Zeiten wurden Proben entnommen und mittels der Ristocetin-mediierten Plättchenaggregation auf ihre von Willebrand Faktor-Aktivität bestimmt.

Bei 4 °C und 25 °C kam es im Beobachtungszeitraum zu keiner Veränderung der Aktivität, bei 37 °C blieb die Aktivität über 24 Std über 80 % und selbst bei 50 °C war über 8 Std keine Veränderung der biologischen Aktivität festzustellen. Gleichzeitig wurde der Antigengehalt mittels ELISA ermittelt. Der Antigengehalt blieb bei allen Lagerungstemperaturen über die gesamte Fleßdauer gleich dem Ausgangswert. Die Stabilitätsuntersuchung wurde ohne die üblichen Proteinstabilisatoren, wie Trägerproteine oder Zucker, durchgeführt.

### BEISPIEL 5

### Lyophilisationsverhalten von rekombinantem von Willebrand Faktor

Ein rekombinanter von Willebrand Faktor wurde, wie in Beispiel 4 beschrieben, formuliert und auf eine Aktivität von 10 E/ml eingestellt. Ohne weiteren Zusatz üblicher stabilisierender Agentien wurde nun gefriergetrocknet und anschließend im Ausgangsvolumen mit Wasser rekonstituiert. Danach wurde neuerlich die Ristocetin-Cofaktor-Aktivität bestimmt. rvWF konnte mit einer Ausbeute von 80 % rekonstituiert werden. Als Vergleichsversuch wurde in Anwesenheit von 0,1 %igem humanem Serumalbumin lyophilisiert, dabei konnten 98 % der Ausgangsaktivität nach Rekonstitution erhalten werden.

### BEISPIEL 6

### Pharmakokinetik der Multimere von rekombinantem von Willebrand Faktor im Schwein.

Für die Untersuchung können von Willebrand-defiziente Tiere, wie z.B. die von Roussi *et al., Brit.J.Haematol.* 90:661-668 1995, beschriebenen homozygoten von Willebranddefizienten Schweine, verwendet werden. In diesem Versuch wurde ein 4 Monate altes, 37 kg schweres, weibliches, homozygotes von Willebrand-defizientes Schwein eingesetzt. Dieses war charakterisiert durch eine Blutungszeit von über 30 Minuten, gemessen nach der Ohrblutungsmethode von Samama et *al., Thromb.Haemostas.* 71:663-669, 1994, und einen von Willebrand Faktor-Plasmaspiegel von unter der Nachweisgrenze, sowohl im Antigen-ELISA als auch in der Ristocetincofaktoraktivität bestimmt. Die Faktor VIII-Aktivität betrug ca. 1 E/ml, gemessen als humaner Faktor VIII im 1-Stufen-Gerinnungstest, 2-Stufen-Gerinnungstest oder chromogenem Faktor VIII-Test (Immunochrom FVIII:C, Immuno).

Unter Narkose wurde dem Schwein eine erfindungsgemäße Präparation, die, wie in Beispiel 2 beschrieben, gewonnen wurde, in einer Dosis von 34 RCoF E/kg Körpergewicht injiziert. Unmittelbar vor der Infusion sowie 30 min, 1 Std, 2 Std, 3 Std, 6 Std, 9 Std, 12 Std, 24 Std, 32 Std und 48 Std nach Infusion wurden Blutproben genommen und daraus Citratplasma hergestellt.

Aus den Plasmaproben wurde die Struktur der von Willebrand Faktor-Multimere durch SDS-Agarosegelelektrophorese im 2 %igen Agarosegel nach der Methode von Ruggeri *et al., Blood* 57:1140-1143, bestimmt. Dabei wurden die von Willebrand Faktor-Multimere durch eine immunenzymatische Färbung nach Aihara *et al., Thromb.Haemostas.* 55:263-267, 1986, sichtbar gemacht. Als primärer Antikörper wurde ein Kaninchen-anti-von Willebrand Faktor-Antiserum (Dakopatts, Glostrup, Denmark) in einer Verdünnung von 1:5000 verwendet. Als sekundärer Antikörper diente ein alkalische Phosphatasekonjugierter, affinitätsgereinigter Ziegen-anti-Kaninchen-IgG H + L Antikörper (Axell, Accurate Chemical and Scientific Corp., NY) in einer Verdünnung von 1:1000. Die Färbung der Proteinbanden erfolgte mittels des Nitroblautetrazolium-chloridbrom-indolyl-phosphatsubstratsystems.

Vor Behandlung mit dem erfindungsgemäßen Präparat konnte kein von Willebrand Faktor im Schwein nachgewiesen werden. Nach Gabe des Präparates zeigte sich eine für den nativen Zustand atypische Struktur eines aus Singuletts bestehenden Multimerenmusters, welches auf einen nicht proteolytisch verdauten von Willebrand Faktor zurückzuführen ist. Diese Struktureigenschaft blieb über den gesamten Beobachtungszeitraum unverändert, d.h. keine proteolytische Degradation des Präparates fand statt. Das Präparat wurde allerdings entsprechend der Pharmakokinetik aus der Zirkulation sukzessive eliminiert. Multimere des niedrigsten Molekulargewichtes blieben bis zu 48 Stunden nach Infusion des Präparates nachweisbar.

Aus den Infusionsexperimenten konnte eine Halbwertszeit des erfindungsgemäßen von Willebrand Faktor von etwa 30 Stunden kalkuliert werden. Als makroskopischen Parameter für die Normalisierung des im defizienten Tier gestörten Gerinnungssystems wurde die Blutungszeit bestimmt, die von über 30 Minuten vor Infusion des von Willebrand Faktors auf ca. 13 Minuten nach Infusion korrigiert werden konnte, wobei dieser Effekt auch noch 32 Stunden nach der Infusion nachweisbar war.

## Patentansprüche

1. Verfahren zur Gewinnung von hochreinem von Willebrand-Faktor, bei dem rekombinanter von Willebrand-Faktor (rvWF) durch eine Anionenaustauschchromatographie an einem Anionenaustauscher vom quaternären Aminotyp in einer Pufferlösung bestehend aus Puffersubstanzen und gegebenenfalls Salz gereinigt wird.

2. Verfahren nach Anspruch 1, dadurch gekennzeichnet, dass der durch die Anionenaustauschchromatographie gereinigte rvWF weiterhin durch eine Affinitätschromatographie an immobilisiertem Heparin in einer Pufferlösung, bestehend aus Puffersubstanzen und gegebenenfalls Salz, gereinigt wird.

3. Verfahren nach Anspruch 2, dadurch gekennzeichnet, dass man ein rvWF-Konzentrat aus zellfreien Kulturüberständen transformierter Zellen reinigt.

4. Verfahren nach einem der Ansprüche 1 bis 3, dadurch gekennzeichnet, dass man als Pufferlösung ein von Stabilisatoren, Aminosäuren und anderen Zusätzen freies Puffersystem verwendet.

5. Verfahren nach einem der Ansprüche 1 bis 4, dadurch gekennzeichnet, dass die Anionenaustauschchromatographie und/oder die Affinitätschromatographie in einem pH-Bereich von 6,0 bis 8,5, und vorzugsweise bei einem pH-Wert von 7,4, durchgeführt werden.

6. Verfahren nach einem der Ansprüche 1 bis 5, dadurch gekennzeichnet, dass der bei der Anionenaustauschchromatographie an den Anionenaustauscher und bei der Affinitätschromatographie an immobilisiertes Heparin gebundene rvWF durch Erhöhung der Salzkonzentration eluiert wird.

7. Verfahren nach einem der Ansprüche 1 bis 6, dadurch gekennzeichnet, dass der quaternäre Anionenaustauscher ein Fraktogel® mit Tentakelstruktur ist.

8. Verfahren nach Anspruch 7, dadurch gekennzeichnet, dass der Anionenaustauscher ein EMD-TMAE-Fraktogel® ist.

9. Verfahren nach einem der Ansprüche 1 bis 8, dadurch gekennzeichnet, dass der rvWF an den Anionenaustauscher bei einer Salzkonzentration von < 270 mM bindet, und bei einer Salzkonzentration > 270 mM, und vorzugsweise bei > 280 mM, eluiert wird.

10. Verfahren nach einem der Ansprüche 2 bis 9, dadurch gekennzeichnet, dass die Affinitätschromatographie an einem Träger mit daran gebundenem Heparin durchgeführt wird, wobei sich bevorzugt AF-Heparin-Toyopear® (Tosohaas), Heparin EMD-Fraktogel® (Merck) oder Heparin Sepharose Fast Flow® (Pharmacia) gleichermassen eignen.

11. Verfahren nach einem der Ansprüche 1 bis 10, dadurch gekennzeichnet, dass der in der Anionenaustauschchromatographie vorgereinigte rvWF an das immobilisierte Heparin bei einer Salzkonzentration < 150 mM bindet und bei einer Salzkonzentration > 150 mM, vorzugsweise bei 200 bis 300 mM, bevorzugter 160 bis 270 mM eluiert wird.

12. Verfahren nach einem der Ansprüche 1 bis 11, dadurch gekennzeichnet, dass als Salze lösliche ein- und zweiwertige Salze verwendet werden.

13. Verfahren nach Anspruch 12, dadurch gekennzeichnet, dass als Salz NaCl verwendet wird.

14. rvWF, der frei von Blutplasmaproteinen, insbesondere frei von Faktor VIII, frei von pathogenen Viren ist und eine hohe Bindungskapazität zu Faktor VIII aufweist, erhalten nach einem Verfahren gemäss einem der Ansprüche 1 bis 13.

15. rvWF nach Anspruch 14, dadurch gekennzeichnet, dass er physiologisch aktiv ist.

16. Verwendung eines rvWF nach Anspruch 14 und/oder 15 zur Herstellung einer pharmazeutischen Zusammensetzung zur Behandlung von Hämophilie A oder Hämophilie A mit einem vWF-Mangel.

17. Verwendung eines rvWF nach Anspruch 14 und/oder 15 zur Herstellung einer pharmazeutischen Zusammensetzung zur Behandlung verschiedener Formen der von Willebrand-Disease.

18. Pharmazeutische Zusammensetzung, dadurch gekennzeichnet, dass sie einen oder mehrere rvWF nach Anspruch 14 und/oder 15 in einem physiologisch annehmbaren Träger enthält.

19. Stabile Präparation enthaltend virus-sicheren rekombinanten von Willebrand Faktor (rvWF), der aus besteht, erhältlich Multimeren mit einer hohen strukturellen Integrität aus einer rvWF-Haltigen Fraktion durch ein chromatographisches Reinigungsverfahren, gemäß einem der Ansprüche 1-13, wobei die Multimeren proteolytisch nicht abgebaut werden.

20. Präparation nach Anspruch 19, dadurch gekennzeichnet, dass der rvWF als nicht-fragmentiertes Protein enthalten ist.

21. Präparation nach Anspruch 19 oder 20, dadurch gekennzeichnet, dass der rvWF nach elektrophoretischer Analyse Multimerenbanden zeigt in Abwesenheit von Satellitenbanden.

22. Präparation nach einem der Ansprüche 19 bis 21, dadurch gekennzeichnet, dass die rvWF-haltige Fraktion erhalten wird aus einer Zellkultur.

23. Präparation nach einem der Ansprüche 19 bis 22, dadurch gekennzeichnet, dass sie als pharmazeutische Zusammensetzung formuliert ist zur Verabreichung an einen Patienten ohne Nebenwirkungen, wie Thrombosebildung, Thrombozytenaktivierung, oder Thrombozytopänie hervorrufen.

24. Pharmazeutische Präparation nach Anspruch 23, welche in Lösung bei Raumtemperatur mindestens 50 Stunden stabil bleibt.

25. Pharmazeutische Präparation nach Anspruch 23 oder 24, dadurch gekennzeichnet, dass sie in Lösung lagerstabil ist.

26. Pharmazeutische Präparation nach einem der Ansprüche 23 bis 25, dadurch gekennzeichnet, dass sie als Infusionspräparat vorliegt.

27. Pharmazeutische Präparation nach einem der Ansprüche 23 bis 26, dadurch gekennzeichnet, dass sie Salze, wie Natriumchlorid und Calciumchlorid, und Aminosäuren, wie Glycin und Lysin, enthält, bei einem pH im Bereich von 6-8.

28. Pharmazeutische Präparation nach einem der Ansprüche 23 bis 27, dadurch gekennzeichnet, dass die rvWF-haltige Fraktion zur Inaktivierung bzw. Abreicherung von Viren behandelt ist.

29. Pharmazeutische Präparation, enthaltend rvWF, gemäß Anspruch 19, der dadurch gekennzeichnet ist, dass er nach Verabreichung an ein Säugetier das Multimerenmuster mit einer Singulettstruktur beibehält.

30. In vitro Verwendung eines rvWF nach Anspruch 14 oder 15 zur Stabilisierung von Faktor VIII, rekombinantem Faktor VIII oder funktionellen Deletionsmutanten von Faktor VIII.

## Claims

1. Method for isolation of highly pure von Willebrand Factor in which recombinant von Willebrand Factor (rvWF) is purified by anion exchange chromatography on an anion exchanger of the quaternary amino type in a buffer solution comprising buffer substances and optionally a salt.

2. Method according to claim 1, characterized in that the rvWF purified by anion exchange chromatography is further purified by affinity chromatography on immobilized heparin in a buffer solution comprising buffer substances and optionally a salt.

3. Method according to claim 2, characterized in that a rvWF concentrate is purified from cell-free culture supernatants of transformed cells.

4. Method according to one of claims 1 to 3, characterized in that a buffer system free of stabilizers, amino acids and other additives is used as a buffer solution.

5. Method according to one of claims 1 to 4, characterized in that the anion exchange chromatography and/or affinity chromatography is carried out at a pH range of 6.0-8.5, and preferably at a pH value of 7.4.

6. Method according to one of claims 1 to 5, characterized in that the rvWF bound in anion exchange chromatography on the anion exchanger and in affinity chromatography on immobilized heparin is eluted by increasing the salt concentration.

7. Method according to one of claims 1 to 6, characterized in that the quaternary anion exchanger is a Fractogel® with tentacle structure.

8. Method according to claim 7, characterized in that the anion exchanger is EMD-TMAE Fractogel®.

9. Method according to one of claims 1 to 8, characterized in that the rvWF is bound to the anion exchanger at a salt concentration ≤ 270 mM, and eluted at a salt concentration > 270 mM and preferably at > 280 mM.

10. Method according to one of claims 2 to 9, characterized in that affinity chromatography is carried out on a carrier with heparin bound thereon, whereby AF-Heparin-Toyopearl® (Tosohaas), Heparin EMD-Fractogel® (Merck) or Heparin Sepharose Fast Flow® (Pharmacia) are preferably equally suitable.

11. Method according to one of claims 1 to 10, characterized in that the rvWF pre-purified in anion exchange chromatography is bound to immobilized heparin at a salt concentration < 150 mM and is eluted at a salt concentration > 150 mM, preferably at 200-300 mM, more preferably 160-270 mM.

12. Method according to one of claims 1 to 11, characterized in that soluble mono- and divalent salts are used as the salt.

13. Method according to claim 12, characterized in that NaCl is used as the salt.

14. rvWF obtained according to a method pursuant to one of claims 1 to 13, which is free from blood plasma proteins, especially free from Factor VIII, free from pathogenic viruses, and possesses a high binding capacity for Factor VIII.

15. rvWF according to claim 14, characterized in that it is physiologically active.

16. Use of a rvWF according to claim 14 and/or 15 for the production of a pharmaceutical composition for treatment of hemophilia A or hemophilia A with a deficiency of vWF.

17. Use of a rvWF according to claim 14 and/or claim 15 for the production of a pharmaceutical composition for treatment of different forms of von Willebrand disease.

18. Pharmaceutical composition characterized in that it comprises one or more rvWF according to claim 14 and/or 15 in a physiologically acceptable carrier.

19. Stable preparation comprising virus-safe recombinant von Willebrand Factor (rvWF), which is comprised of multimers with a high structural integrity, obtainable from a rvWF containing fraction by a chromatographic purification method according to one of claims 1 to 13, whereby the multimers are not proteolytically degraded.

20. Preparation according to claim 19, characterized in that the rvWF is contained as a nonfragmented protein.

21. Preparation according to claim 19 or 20, characterized in that the rvWF has multimer bands in the absence of satellite bands after electrophoresis analysis.

22. Preparation according to one of claims 19 to 21, characterized in that the rvWF-containing fraction is obtained from a cell culture.

23. Preparation according to one of claims 19 to 22, characterized in that it is formulated as a pharmaceutical composition for administration to patients without causing side-effects such as the formation of thrombi, thrombocyte activation or thrombocytopenia.

24. Pharmaceutical preparation according to claim 23, which remains stable in solution at room temperature for at least 50 hours.

25. Pharmaceutical preparation according to claim 23 or 24, characterized in that it is storable in solution.

26. Pharmaceutical preparation according to one of claims 23 to 25, characterized in that it is present as an infusion preparation.

27. Pharmaceutical preparation according to one of claims 23 to 26, characterized in that it comprises salts, such as sodium chloride and calcium chloride, and amino acids such as glycine and lysine at a pH in the range of 6-8.

28. Pharmaceutical preparation according to one of claims 23 to 27, characterized in that the rvWF-containing fraction is treated for inactivation and/or depletion of viruses.

29. Pharmaceutical preparation comprising rvWF according to claim 19 which is characterized in that it maintains the multimer pattern with a singlet structure after administration to a mammal.

30. In vitro use of rvWF according to claim 14 or 15 for stabilization of Factor VIII, recombinant Factor VIII or functional deletion mutants of Factor VIII.

## Revendications

1. Procédé de préparation du facteur Willebrand sous une forme très pure, dans lequel un facteur Willebrand de recombinaison (FWr) est purifié par une chromatographie d'échange d'anions sur un échangeur d'anions portant des groupes du type amino quaternaire, dans une solution tampon contenant des substances tampon et, le cas échéant, du sel.

2. Procédé selon la revendication 1, caractérisé en ce que le FWr purifié par la chromatographie sur un échangeur d'anions est ensuite purifié par une chromatographie d'affinité sur de l'héparine immobilisée, dans une solution tampon contenant des substances tampon et, le cas échéant, du sel.

3. Procédé selon la revendication 2, caractérisé en ce que l'on purifie un concentré de FWr d'un surnageant exempt de cellules, provenant d'une culture de cellules transformées.

4. Procédé selon l'une des revendications 1 à 3, caractérisé en ce que la solution tampon est un système tampon exempt de stabilisants, d'acides aminés et d'autres additifs.

5. Procédé selon l'une des revendications 1 à 4, caractérisé en ce que l'on effectue la chromatographie d'échange d'anions et/ou la chromatographie d'affinité dans une plage de pH allant de 6,0 à 8,5 et, de préférence, à une valeur de pH de 7,4.

6. Procédé selon l'une des revendications 1 à 5, caractérisé en ce que l'on élue le FWr lié, dans la chromatographie d'échange d'anions à l'échangeur d'anions et dans la chromatographie d'affinité, à l'héparine immobilisée, en augmentant la concentration saline.

7. Procédé selon l'une des revendications 1 à 6, caractérisé en ce que l'échangeur d'anions quaternaire est la résine Fraktogel ® avec une structure tentaculaire.

8. Procédé selon la revendication 7, caractérisé en ce que l'échangeur d'anions est une résine EMD-TMAE-Fraktogel ®.

9. Procédé selon l'une des revendications 1 à 8, caractérisé en ce que le FWr est fixé sur l'échangeur d'anions à une concentration saline ≤ 270 mM et élué à une concentration saline > 270 mM et, de préférence > 280 mM.

10. Procédé selon l'une des revendications 2 à 9, caractérisé en ce que la chromatographie d'affinité est effectuée sur un support portant de l'héparine liée, la préférence allant indifféremment aux résines AF-Heparin-Toyopearl ® (Tosohaas), Heparin EMD-Fraktogel ® (Merck) ou Heparin Sepharose Fast Flow ® (Pharmacia).

11. Procédé selon l'une des revendications 1 à 10, caractérisé en ce que le FWr prépurifié par chromatographie sur échangeur d'anions est fixé à l'héparine immobilisée à une concentration saline < 150 mM et élué à une concentration saline > 150 mM, de préférence entre 200 et 300 mM ou, encore mieux, entre 160 et 270 mM.

12. Procédé selon l'une des revendications 1 à 11, caractérisé en ce que le sel utilisé est un sel soluble monovalent ou divalent.

13. Procédé selon la revendication 12, caractérisé en ce que le sel utilisé est le NaCl.

14. FWr exempt de protéines du plasma sanguin, en particulier exempt de facteur VIII, de virus pathogènes et ayant une capacité de fixation élevée au facteur VIII, obtenu par un procédé selon l'une des revendications 1 à 13.

15. FWr selon la revendication 14, caractérisé en ce qu'il est actif physiologiquement.

16. Utilisation d'un FWr selon la revendication 14 et/ou la revendication 15 pour obtenir une composition pharmaceutique pour traiter l'hémophilie A, ou l'hémophilie A accompagnée d'une déficience en FWr.

17. Utilisation d'un FWr selon la revendication 14 et/ou la revendication 15 pour obtenir une composition pharmaceutique pour traiter différentes formes de la maladie de von Willebrand.

18. Composition pharmaceutique caractérisée en ce qu'elle contient un ou plusieurs FWr selon la revendication 14 et/ou la revendication 15, dans un vecteur acceptable sur le plan physiologique.

19. Préparation stable contenant un facteur Willebrand de recombinaison (FWr) inaccessible aux virus, qui est constitué de multimères avec une intégrité structurelle élevée, qui peut être obtenu à partir d'une fraction contenant du FWr par un procédé de purification par chromatographie, selon l'une quelconque des revendications 1 - 13 et dont les multimères ne subissent pas de dégradation protéolytique.

20. Préparation selon la revendication 19, caractérisée en ce que le FWr est présent sous la forme d'une protéine non fragmentée.

21. Préparation selon la revendication 19 ou la revendication 20, caractérisée en ce que le FWr après analyse par électrophorèse, présente des bandes multimères sans bandes satellites.

22. Préparation selon l'une des revendications 19 à 21, caractérisée en ce que la fraction contenant le FWr provient d'un milieu de culture de cellules.

23. Préparation selon l'une des revendications 19 à 22, caractérisée en ce qu'elle est formulée en tant que composition pharmaceutique pour traiter un patient, sans effets secondaires, tels que la formation de thromboses, l'activation de thrombocytes ou l'apparition d'une thrombopénie.

24. Préparation pharmaceutique selon la revendication 23, qui reste stable en solution à température ambiante pendant au moins 50 heures.

25. Préparation pharmaceutique selon la revendication 23 ou la revendication 24, caractérisée en ce que la solution est stable en stockage.

26. Préparation pharmaceutique selon une des revendications 23 à 25, caractérisée en ce qu'elle se présente comme préparation pour perfusion.

27. Préparation pharmaceutique selon l'une des revendications 23 à 26, caractérisée en ce qu'elle contient des sels, tels que le chlorure de sodium et le chlorure de calcium et des acides aminés, comme par exemple la glycine et la lysine et que son pH est dans la plage de 6 - 8.

28. Préparation pharmaceutique selon l'une des revendications 23 à 27, caractérisée en ce que la fraction contenant le FWr est traitée pour inactiver ou appauvrir les virus.

29. Préparation pharmaceutique contenant du FWr selon la revendication 19, caractérisée en ce qu'après administration à un mammifère, le motif de multimères à structure de singulets est conservé.

30. Utilisation in vitro d'un FWr selon la revendication 14 ou la revendication 15 pour stabiliser un facteur VIII, un facteur de recombinaison VIII ou des mutants du facteur VIII par délétion, qui sont fonctionnels.
